# EUROPEAN PATENT SPECIFICATION

(11) **EP 1 395 322 B1**
(45) Date of publication and mention of the grant of the patent: **14.09.2005**
(21) Application number: 02724487.0
(22) Date of filing: 13.05.2002
(51) Int. Cl.: A61M 16/01

(54) **INHALATION HANDSET**
HANDTEIL EINES INHALATORS
PIECE A MAIN POUR INHALATION

(30) Priority: 19.05.2001 GB 0112264
(43) Date of publication of application: 10.03.2004
(73) Proprietor: INTERSURGICAL LIMITED, Berkshire RG41 2RZ (GB)
(72) Inventor: PITTAWAY, Alan Kenneth, High Wycombe, Buckinghamshire HP13 5QP (GB)
(74) Representative: Jones, Stephen Anthony
(86) International application number: PCT/GB2002/002193
(87) International publication number: WO 2002/094360

(56) References cited:
- EP-A- 0 604 399
- WO-A-97/48433
- US-A- 4 048 993
- US-A- 4 463 755
- US-A- 5 871 011

## Description

This invention relates to an inhalation handset, that is to say a device intended to be connected to a supply of gas, which may be held by a user and employed by the user for the self-administration of that gas, and by which gas exhaled by the user may be collected and transmitted to a remote gas scavenging system. A particular application of such a system is in the self-administration of mild anaesthetic gases by expectant mothers undergoing labour and childbirth.

Document US 4 048 993 discloses an inhalation handset with the features of the preamble of claim 1.

According to the invention, an inhalation handset according to claim 1 is provided.

The inhalation handset according to the invention is advantageous principally because the filter is built into the handset. This means that a separate filter is not required in a breathing circuit of which the inhalation handset forms a component. There is therefore less chance of error by the medical staff when constructing the breathing circuit. In addition, the handset and breathing system may be completely disposable, thereby minimising cross-infection, transmission of prions, etc.

It is preferred that the supply conduit and the exhaust conduit should be coaxial, such that a single co-axial pipe or tube serves for both supply of the gas, such as the anaesthetic known as Entonox, and removal of exhaled gas to a gas scavenging system. In such a case, the inner lumen of the co-axial pipe is preferably the supply conduit and the outer lumen is preferably the exhaust conduit.

In this preferred arrangement, scavenging of the exhaled gas is automatic as there is no need to connect an additional exhaust pipe to the handset or breathing system. The integration of the supply and exhaust pipes will therefore minimise possible errors by medical staff when constructing the breathing circuit, such as not connecting an exhaust pipe. This prevents gas, such as the anaesthetic known as Entonox, being vented into the immediate area.

The filter is preferably of a woven material, eg a glass microfibre, and is intended to prevent contamination of the gas supply system with any infective agents exhaled by the patient.

The handset is preferably elongate in shape and is typically generally tubular. The filter preferably extends from a position between the first and second valve means to an internal surface of the handset such that gas flowing from the supply conduit through the first valve means must pass through the filter to reach the mouthpiece. The filter is preferably generally rectangular in shape and is preferably disposed at a relatively small angle to the longitudinal axis of the handset. The flow of gas between the supply conduit and the mouthpiece will generally be in a direction that is parallel to the longitudinal axis of the handset, and hence at a small angle to surface of the filter.

Such a filter arrangement is advantageous because the filter, being inclined to the flow of gas from the supply conduit to the mouthpiece, presents a relatively large surface area to the gas. This arrangement therefore ensures effective filtration performance without excessive resistance to flow of gas, and eliminates the need for excessive suction to be applied at the mouthpiece in order for gas to be inhaled from the supply conduit. This is particularly important because a user who is in pain, such as a woman during childbirth, may not be able to provide much suction at the mouthpiece.

The filter is arranged at an angle of less than 45° to the direction of flow of gas, preferably less than 30°, but greater than 0°, more preferably greater than 5°.

In preferred embodiments, the handset and the associated conduits together constitute a fully disposable unit, intended to be discarded after a single use.

According to another aspect of the invention, there is provided an anaesthetic apparatus comprising an inhalation handset according to claim 1.

The invention will now be described in greater detail, by way of illustration only, with reference to the accompanying drawings, in which
Figure 1 is a perspective view of a first embodiment of an inhalation handset according to the invention;
Figure 2 is a cut-away view of the handset of Figure 1, showing the internal construction;
Figure 3 is a view similar to Figure 1 of a second embodiment of an inhalation handset according to the invention; and
Figure 4 is a view similar to Figure 4 of the handset of Figure 3.

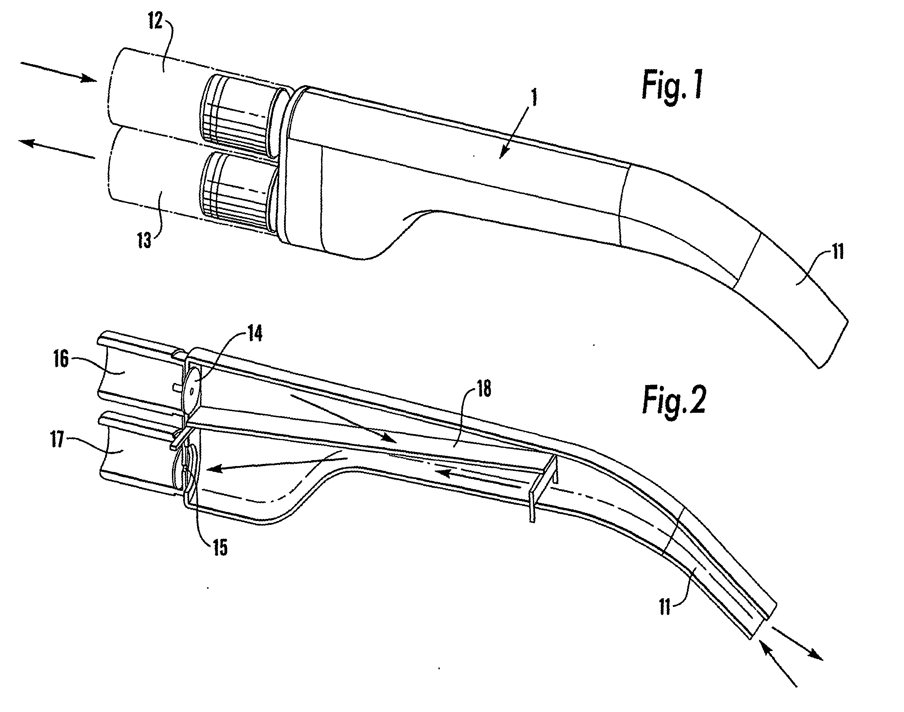

Referring first to Figures 1 and 2, a first embodiment of an inhalation handset according to the invention is generally designated 1. The handset is intended for the delivery of a mild anaesthetic gas such as that known as Entonox, as is commonly used by expectant mothers in the course of labour and childbirth. The handset 1 is moulded in plastics material.

The handset is shaped and dimensioned to be readily held by the user, and one end (the right hand end as viewed in the drawings) is formed into the shape of a spout or mouthpiece 11. The other end of the handset 1 is formed with connectors 16,17 (see Figure 2) by which the handset 1 is connected respectively to a supply gas tube 12 and an exhaust tube 13. The supply gas tube 12 and the exhaust tube 13 both take the form of flexible hoses of plastics material, the ends of which are fitted over, and bonded to, the respective connectors 16,17.

The other end of the gas supply tube 12 is connected to a supply of Entonox/air gas, and the other end of the exhaust tube 13 is connected to a conventional gas scavenging system (which operates at reduced pressure).

As can be seen from Figure 2, the connections between the handset 1 and the supply gas tube 12 and the exhaust tube 13 are both fitted with one-way valves 14,15. The valve 14 to which the supply gas tube 12 is connected permits flow of gas into the handset 1, while the valve 15 to which the exhaust tube 13 is fitted permits gas to flow out of the handset 1.

The valves 14,15 take the form of flexible discs of an elastomeric material. The discs are anchored at their centre, but are able to flex towards the interior of the handset 1 (in the case of the supply valve 14) and towards the exhaust tube 13 (in the case of the exhaust valve 15). Flexing of the discs in the opposite direction is prevented by valve seats constituted by appropriately shaped parts of the plastic moulding of the handset 1.

As can also be seen from Figure 2, a filter medium 18 is disposed within the interior of the handset 1, and extends from a point between the two one-way valves 14,15 to the upper (as viewed in the drawings) internal surface of the handset 1. The filter medium 18 thus divides the interior of the handset 1 into two compartments. The filter medium is of a glass microfibre material.

In use, the user holds the handset and raises the mouthpiece 11 to her mouth whenever she desires to take in the Entonox/air mixture. By inhaling, the user draws such gas through the one way valve 14 and through the filter medium 18, and inhales it. Expired air is exhaled into the handset and passes beneath the filter medium 18 and through the one-way valve 15 into the exhaust tube 13, from where the exhaled air is collected in the scavenging system. The filter medium 18 prevents potentially infective agents exhaled by the user reaching, and contaminating, the gas supply system.

The handset 1 and associated tubes 12,13 are intended to be used only once, being entirely disposable after use by one user. Since the filter medium 18 is built into the handset 1, it is not necessary for the filter to be fitted to the system by medical or technical staff prior to use. Because the exhaust tube 13 is prebonded to the handset 1, there is also no danger of the handset 1 being used without the exhaust tube 13 being in place, thereby eliminating the danger of Entonox gas (as well as any infectious agents exhaled by the patient) being vented into the immediate area.

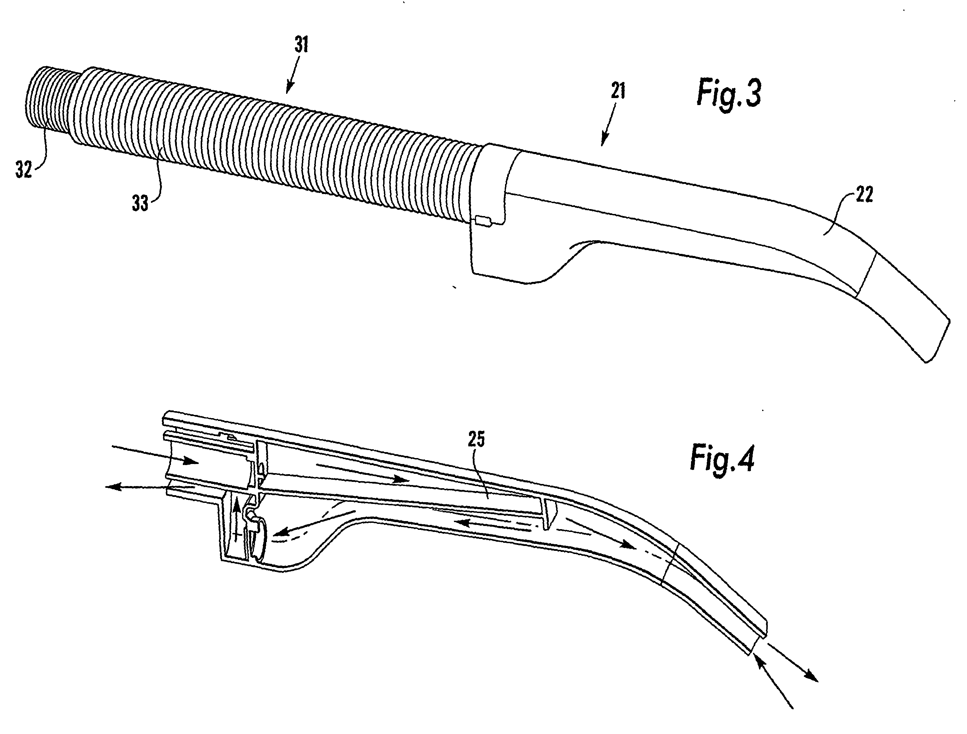

Referring now to Figures 3 and 4, the second embodiment of the invention is similar to the first embodiment in that the handset 21 is of generally similar form, being configured in such a way as to be easily held by the user and having a mouthpiece 22. Where the second embodiment differs from the first is in that the gas supply tube and exhaust tube are embodied in a single, co-axial pipe 31.
The inner lumen 32 of the pipe 31 serves as the gas supply tube and the outer lumen 33 serves as the exhaust tube. The geometry of the left-hand (as viewed in the drawings) end of the handset is altered correspondingly, to permit communication between the two chambers within the interior of the handset 21 (defined by the filter medium 25 that partitions the internal volume of the handset 21) and the respective parts of the co-axial pipe 31.

The second embodiment is used in a similar manner to the first, but offers the further advantage that the co-axial pipe is more compact, there being no cumbersome second limb of the breathing circuit that could impede medical or midwifery personnel attending the user. Again, the handset 21 and coaxial pipe 31 are fitted together and are intended to be fully disposable.

## Claims

1. An inhalation handset (1,21) adapted to be held by a user and formed with a mouthpiece (11,22) by which gas can be inhaled by the user from the handset and exhaled by the user into the handset, the handset being adapted to be coupled to a supply conduit (12,32) and an exhaust conduit (13,33), and comprising first valve means (14) permitting flow of gas from the supply conduit into the handset and second valve means (15) permitting flow of gas from the handset into the exhaust conduit, **characterised in that** a filter (18,25) is disposed within the handset so as to partition the internal volume of the handset, the filter being interposed in the flow of gas from the supply conduit to the mouthpiece and arranged at an angle of less than 45° to the direction of flow of gas.

2. An inhalation handset as claimed in Claim 1, wherein the supply conduit and the exhaust conduit are coaxial such that a single co-axial pipe or tube serves for both supply of the gas and removal of exhaled gas.

3. An inhalation handset as claimed in Claim 2, wherein the inner lumen of the co-axial pipe is the supply conduit and the outer lumen is the exhaust conduit.

4. An inhalation handset as claimed in any preceding claim, wherein the gas inhaled by the user is an anaesthetic.

5. An inhalation handset as claimed in any preceding claim, wherein the filter is a woven material and is intended to prevent contamination of the gas supply system with any infective agents exhaled by the patient.

6. An inhalation handset as claimed in any preceding claim, wherein the handset and the associated conduits together constitute a fully disposable unit, intended to be discarded after a single use.

7. An inhalation handset as claimed in any preceding claim, wherein the handset is elongate in shape and generally tubular.

8. An inhalation handset as claimed in any preceding claim, wherein the filter extends from a position between the first and second valve means to an internal surface of the handset such that gas flowing from the supply conduit through the first valve means must pass through the filter to reach the mouthpiece.

9. An inhalation handset as claimed in any preceding claim, wherein the filter is arranged at an angle of less than 30° to the direction of flow of gas.

10. An inhalation handset as claimed in any preceding claim, wherein the filter is arranged at an angle of greater than 0° to the direction of flow of gas.

11. An inhalation handset as claimed in Claim 11, wherein the filter is arranged at an angle of greater than 5° to the direction of flow of gas.

12. Anaesthetic apparatus comprising an inhalation handset according to any preceding claim.

## Patentansprüche

1. Ein Handgerät zur Inhalation (1,21), so angepasst, um von dem Benutzer gehalten zu werden und aus einem Mundstück (11,22) gebildet, über das vom Benutzer Gas von dem Handgerät eingeatmet werden kann und vom Benutzer in das Handgerät ausgeatmet werden kann, wobei das Handgerät angepasst ist, um mit einem Zuführungs-Kanal (12,32) und mit einem Ausatmungs-Kanal (13,33) verbunden zu werden und ein erstes Ventilelement (14) umfasst, das das Strömen des Gases von dem Zuführungs-Kanal zu dem Handgerät gestattet, und ein zweites Ventilelement (15), das das Strömen des Gases von dem Handgerät in den Ausatmungs-Kanal gestattet,
**dadurch gekennzeichnet, dass** ein Filter (18,25) so im Handgerät angebracht ist, um den inneren Rauminhalt des Handgeräts aufzuteilen, wobei der Filter im Gasstrom von dem Zuführungs-Kanal zu dem Mundstück eingefügt ist, und mit einem Winkel von weniger als 45° in Strömungsrichtung des Gases angeordnet ist.

2. Ein Handgerät zur Inhalation nach Anspruch 1, wobei der Zuführungs-Kanal und der Ausatmungs-Kanal koaxial sind, so dass eine einzige ko-axiale Röhre oder ein Schlauch sowohl zur Zuführung von Gas, als auch zur Beseitigung des ausgeatmeten Gas dient.

3. Ein Handgerät zur Inhalation nach Anspruch 2, wobei das innere Lumen der ko-axialen Röhre der Zuführungs-Kanal ist, und das äußere Lumen der Ausatmungs-Kanal ist.

4. Ein Handgerät zur Inhalation nach einem der vorhergehenden Ansprüche, wobei das von dem Benutzer eingeatmete Gas ein narkotisches Gas ist.

5. Ein Handgerät zur Inhalation nach einem der vorhergehenden Ansprüche, wobei der Filter aus einem Gewebematerial besteht, und dazu gedacht ist, eine Kontaminierung des Gas-Zuführungs-Systems mit infektiösen Wirkstoffen, die vom Patienten ausgeatmet werden, zu verhindern.

6. Ein Handgerät zur Inhalation nach einem der vorhergehenden Ansprüche, wobei das Handgerät und die damit verbundenen Kanäle eine komplette Einweg-Einheit darstellen, die dazu gedacht ist, nach einmaligem Gebrauch weggeworfen zu werden.

7. Ein Handgerät zur Inhalation nach einem der vorhergehenden Ansprüche, wobei das Handgerät in seiner Form verlängert ist und im Allgemeinen röhrenförmig.

8. Ein Handgerät zur Inhalation nach einem der vorhergehenden Ansprüche, wobei sich der Filter von einer Stelle zwischen dem ersten und dem zweiten Ventilelement zu einer inneren Oberfläche des Handgerätes erstreckt, so dass das von dem Zuführungs-Kanal durch das erste Ventilelement fließende Gas den Filter passieren muss, um das Mundstück zu erreichen.

9. Ein Handgerät zur Inhalation nach einem der vorhergehenden Ansprüche, wobei der Filter mit einem Winkel von weniger als 30° in Strömungsrichtung des Gases angeordnet ist.

10. Ein Handgerät zur Inhalation nach einem der vorhergehenden Ansprüche, wobei der Filter mit einem Winkel von mehr als 0° in Strömungsrichtung des Gases angeordnet ist.

11. Ein Handgerät zur Inhalation nach Anspruch 11, wobei der Filter mit einem Winkel von mehr als 5° in Strömungsrichtung des Gases angeordnet ist.

12. Narkose-Apparat, der ein Handgerät zur Inhalation nach einem der vorhergehenden Ansprüche umfasst.

## Revendications

1. Un inhalateur manuel (1,21), prévu pour être maintenu par un utilisateur, composé d'une adaptation buccale (11,22) à travers laquelle le gaz est inhalé par l'utilisateur à partir de l'inhalateur et expiré par l'utilisateur dans l'inhalateur ; l'inhalateur étant prévu pour être monté sur un conduit d'alimentation (12,32) et sur un conduit d'évacuation (13,33), et comprenant un premier système de vannes (14) permettant la circulation du gaz depuis le conduit d'alimentation vers l'inhalateur et un second système de vannes (15) permettant la circulation du gaz depuis l'inhalateur dans le conduit d'évacuation,
**caractérisé en ce qu'**un filtre (18,25) est disposé à l'intérieur de l'inhalateur de sorte à cloisonner le volume interne de l'inhalateur manuel, le filtre étant disposé sur la trajectoire du gaz circulant depuis le conduit d'alimentation vers l'adaptation buccale et fixé selon un angle inférieur à 45° dans le sens de la circulation du gaz.

2. Un inhalateur manuel selon la revendication 1, dans lequel le conduit d'alimentation et le conduit d'évacuation sont coaxiaux de sorte qu'un seul tuyau ou tube coaxial est nécessaire pour l'alimentation du gaz et l'évacuation du gaz exhalé.

3. Un inhalateur manuel selon la revendication 2, dans lequel le lumen interne du tuyau coaxial est le conduit d'alimentation et dans lequel le lumen externe est le conduit d'évacuation.

4. Un inhalateur manuel selon l'une quelconque des revendications précédentes, dans lequel le gaz inhalé par l'utilisateur est un anesthésique.

5. Un inhalateur manuel selon l'une quelconque des revendications précédentes, dans lequel le filtre est un matériau tissé et est prévu pour empêcher que le système d'alimentation en gaz ne soit contaminé par un agent infectieux rejeté par le patient.

6. Un inhalateur manuel selon l'une quelconque des revendications précédentes, dans lequel l'inhalateur ainsi que les conduits associés constituent une unité entièrement jetable, prévue pour ne servir qu'une seule fois.

7. Un inhalateur manuel selon l'une quelconque des revendications précédentes, dans lequel l'inhalateur possède une forme allongée et généralement tubulaire.

8. Un inhalateur manuel selon l'une quelconque des revendications précédentes, dans lequel le filtre se déplace d'une position située entre le premier et le deuxième système de vannes vers la surface interne de l'inhalateur manuel de sorte que le gaz circulant depuis le conduit d'alimentation à travers le premier système de vannes doivent passer à travers le filtre pour atteindre l'adaptation buccale.

9. Un inhalateur manuel selon l'une quelconque des revendications précédentes, dans lequel le filtre est fixé selon un angle inférieur à 30° dans le sens de la circulation du gaz.

10. Un inhalateur manuel selon l'une quelconque des revendications précédentes, dans lequel le filtre est fixé selon un angle supérieur à 0° dans le sens de la circulation du gaz.

11. Un inhalateur manuel selon la revendication 11, dans lequel le filtre est fixé selon un angle supérieur à 5° dans le sens de la circulation du gaz.

12. Appareil pour anesthésie comprenant an inhalateur manuel selon l'une quelconque des revendications précédentes.
